# EUROPEAN PATENT SPECIFICATION

(11) **EP 4 151 265 B1**
(45) Date of publication and mention of the grant of the patent: **13.08.2025**
(21) Application number: 22194284.0
(22) Date of filing: 07.09.2022
(51) Int. Cl.: A61M 25/09

(54) **NEURO ACCESS GUIDE WIRE**
NEUROZUGANGSFÜHRUNGSDRAHT
FIL DE GUIDAGE D'ACCÈS NEUROLOGIQUE

(30) Priority: 08.09.2021 US 202163241881 P
(43) Date of publication of application: 22.03.2023
(73) Proprietor: Neuravi Limited, Galway H91 K5YD (IE)
(72) Inventor: KEATING, Karl, Galway, H91 K5YD (IE); VALE, David, Galway, H91 K5YD (IE)
(74) Representative: Carpmaels & Ransford LLP

(56) References cited:
- WO-A1-2022/120068
- CN-A- 107 362 437
- CN-B- 102 921 094
- JP-A- 2010 227 136
- US-A1- 2006 064 036

## Description

### Field of the Invention

The present invention relates to guidewire that can be used to hold or expand a large bore catheter while accessing the neurovascular.

### Background

Guidewires are known in the art along with large bore catheters. However, passing a large bore catheter over a guidewire to its location within the neurovascular can be challenging given the size differential. Typically, an intermediate catheter is used that is sized between the devices to help with transition or opening the distal end of the large bore catheter. Using a second catheter can be a waste of resources and also time as the user needs to advance the intermediate catheter prior to the large bore catheter.

US2006/064036A1 describes a wire guide having a wire core and a braided sheath. The wire core includes a proximal end and distal end, wherein the braided sheath is attached to the distal end of the wire core and serves as a flexible pulling section. The braided sheath is woven of a plurality of strands and may be made of various material based on the application, such as stainless steel, a shape memory alloy, or a radiopaque material. The wire guide has a flexible tip at the proximal end opposite the flexible pulling section. A stiff section is provided between the flexible tip and the flexible pulling section to allow manipulation of the wire guide through a body lumen. Proximate the distal end of the wire core a tapered section is provided to increase flexibility of the wire guide toward the distal end. The braided sheath is received over and attached to the wire core. In addition, a shoulder is provided in the wire core providing a smooth transition from the wire core to the braided section. The braided sheath extends from the shoulder beyond the distal end of the wire core.

CN102921094B describes an intravascular expansion guide wire and a preparation method thereof. The technical problem to be solved is to pre-expand the stenotic part of the blood vessel, so that the balloon with the preloaded bracket can pass through the stenotic lesion part of the blood vessel. The intravascular expansion guide wire adopted in the present invention is provided with a core wire, the middle part of the core wire is an expansion part, and the outer diameter of the expansion part is larger than the outer diameter of the core wire. The preparation method of the present invention comprises the following steps: 1. making a guide wire head; 2. making a core wire; and 3. assembling a guide wire head on the core wire to obtain an intravascular expansion guide wire. Compared with the prior art, the present invention pre-expands the stenotic section of the blood vessel by using the part with the largest diameter of the expansion guide wire, so that the inner cavity of the stenotic section becomes larger, thereby facilitating the passage of the balloon through the stenotic section.

CN107362437A describes a guide wire. The guide wire comprises a far end, a near end and a core wire, the core wire is positioned in a guide wire body and extends from the near end to the far end, a dilating portion is arranged on the core wire, the diameter of the dilating portion is larger than that of the core wire, and a safety net is arranged at a head end, positioned at the far end, of the core wire and is fixedly connected with the head end. The invention further discloses a preparation method of the guide wire. By additionally arranging the safety net at the head end, positioned at the far end, of the core wire, loss of torsion/torque transmission of the guide wire is reduced, and softness and non-deformability of the far end of the guide wire are guaranteed while the phenomenon of 'tail whipping' is eliminated; by arranging the dilating portion on the guide wire, a narrow portion can be pre-dilated by a radial support effect, and an inner cavity of the narrow portion can be dilated to allow a balloon to pass through conveniently. The dilating portion of the pre-dilating guide wire is perfectly round, smooth and soft and cannot cause wounds to blood vessels.

WO2022/120068A1 describes a guidewire 100 including a distal section 101, a proximal section 103 and a middle section 102 disposed between the distal section and the proximal section, wherein the middle section has a flexural stiffness that is greater than a flexural stiffness of both of the distal section and the proximal section. The distal section is configured for insertion into a vasculature of a patient. A diameter of the middle section may be greater than a diameter of the distal section. The guidewires may include a tapered distal transition portion disposed between the distal section and the middle section and a solid core wire extending a length of the guidewire, the solid core wire including a first diameter extending along the distal section, a second diameter extending along the proximal section, and a third diameter extending along the middle section, wherein the third diameter is greater than the first and second diameters.

JP2010227136A describes a guide wire 10 which includes a first distal end part 20 as one distal end area and a second distal end part 30 as the other distal end area on the opposite side to the first distal end part 20. The proximal end of the first distal end part 20 and the proximal end of the second distal end part 30 are bonded to each other, and the insert direction of the guide wire 10 is changed to insert both the distal end of the first distal end part 20 and the distal end of the second distal end part 30 in a lumen such as a blood vessel. Since the outside diameter of the first distal end part 20 is smaller than that of the second distal end part 30, the first distal end part 20 has higher flexibility than the second distal end part 30.

### Summary

Examples of the present invention of an access guide wire that can have a section that includes larger outer diameter (OD) portion to bridge gap between guidewire OD of 0.014 inch or 0.022 inch and a large bore catheter inner diameter (ID) of 1.778 mm (0.070 inch). These sizes are important when accessing the distal neurovascular, there is a gap between the ID of large bore catheters and guidewire ODs used in gaining access to distal cerebral vessels such as the ICA and the M1/M2.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1 illustrates a side view of the access wire of the present invention.
Figure 2 illustrates a side view of the access wire with a deflected distal end.
Figure 3 illustrates a cross-section view of another example of the access wire.
Figure 4 illustrates a cross-section view of further example of the access wire.
Figure 5 illustrates a cross-section view of yet another example of the access wire.
Figure 6 illustrates a side view of another example of the access wire.
Figure 7 illustrates a partial cross-section view of the access wire inside a large bore catheter.
Figure 8 illustrates a cross-section along line X-X of Figure 7.

### Detailed Description

Specific examples of the present invention are now described in detail with reference to the Figures, where identical reference numbers indicate elements which are functionally similar or identical. Accessing the various vessels within the vascular, whether they are coronary, pulmonary, or cerebral, involves well-known procedural steps and the use of a number of conventional, commercially-available accessory products. These products, such as angiographic materials, rotating hemostasis valves, and large and intermediate catheters are widely used in laboratory and medical procedures. When these products are employed in conjunction with the system and methods of this invention in the description below, their function and exact constitution are not described in detail. While the description is in many cases in the context of neurovascular treatments, the systems and methods may be used for other procedures and in other body passageways as well.

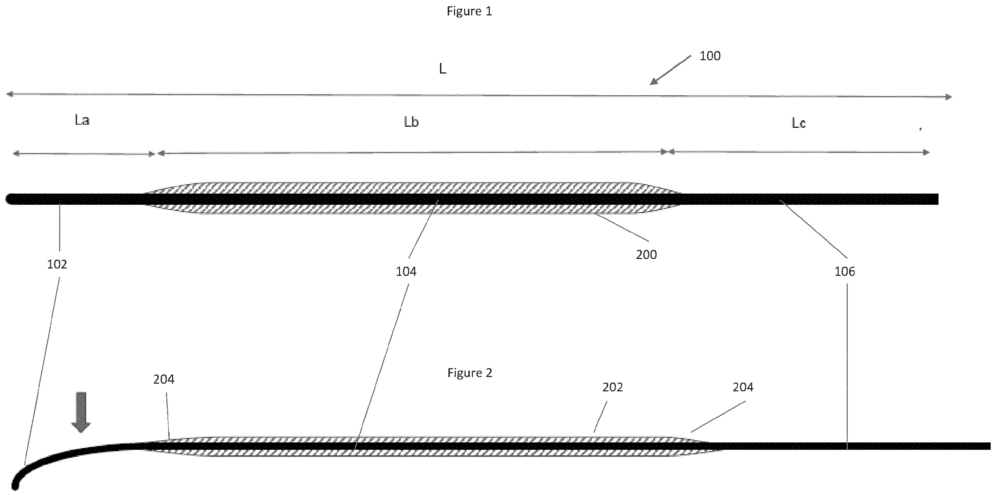

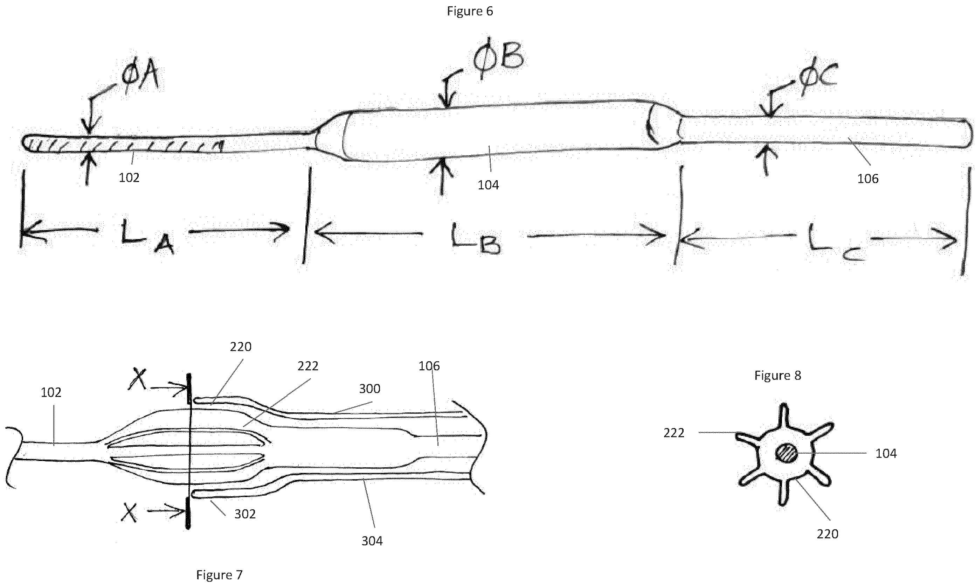

Figure 1 illustrates an example of an access guidewire 100 of the present invention. A typical prior art guidewire has a uniform diameter across its length. The access guidewire 100 has a length L, a distal portion 102, and intermediate portion 104, and a proximal portion 106. The distal portion 102 can have a length La, the intermediate portion 104 has a length Lb and the proximal portion has a length Lc. The intermediate portion 104 can have an increased diameter ΦB in relation to the distal portion diameter ΦA and the proximal portion diameter ΦC (see Figure 6). In Figures 1 and 2 the distal portion diameter ΦA and the proximal portion diameter ΦC can be equal while the intermediate portion diameter ΦB is larger. The increased diameter portion 200 can be same material as the rest of the guidewire 100, just formed with a larger circumference/diameter.

Figure 2 illustrates that the distal portion 102 can retain its flexibility along its length La as is typical for a guidewire. The increased diameter portion 200 can have reduced flexibility or retain flexibility consistent with the distal portion 102. In this example, the proximal portion 106 can be the portion proximal the increased diameter portion 200 and extend to the operator. The flexibility of the proximal portion 106 can mirror that of a typical guidewire or have differential flexibility along its length as may be necessary to deliver it to the neurovascular region of a patient.

Figure 1 illustrates an example where the intermediate portion 104 and the increased diameter portion 200 are made of the same material. Figures 2, 3, 4, and 5 illustrate examples where the increased diameter portion 200 can be separately provided on the intermediate portion 104.

Figures 2, 3, 4, and 5, illustrate an example where the access guidewire 100 has a uniform diameter across the distal portion 102, the intermediate portion 104, and the proximal portion 106. Instead, as in Figure 2, the increased diameter portion 202 can be formed over the intermediate portion 104. In one example, the increased diameter portion 202 can be a soft reflowed jacket over the guidewire 100 with tapered ends 204.

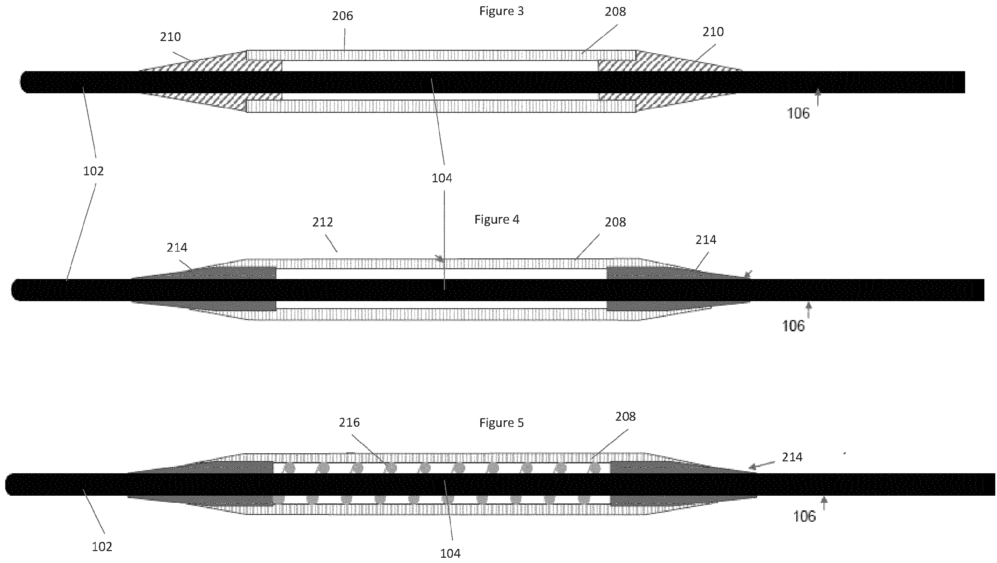

Figure 3 illustrates that the increased diameter portion 206 can have an outer tube 208 or composite tube which can also have a coil/braid support. The tapered ends 210 can be the adhesive bond that holds the outer tube 208 in place and is filleted to allow for a smooth transition. In one example, the outer tube 208 can be made of Pebax 25D or 35D or Neusoft Jacket 42A/62A.

Figure 4 illustrates a two-tube example for the increased diameter portion 212. Here the outer tube 208 is placed over an inner tube 214 and the inner and outer tubes 214, 208 are reflowed to form the increased diameter portion 212. In this example, the outer tube 208 can be made of Pebax 25D or 35D while the inner tube 214 can be made of Neusoft 42A/62A.

Figure 5 illustrates a variant example of Figures 3 and 4, here a coil 216 is placed inside the outer tube 208 to provide strength and kink resistance. Note that in the variant of increased diameter portion 212 of Figure 4, the outer tube 208 and the inner tube 214 are reflowed to have tapered profile. The inner coil 216 offers kink resistance with thin flexible outer tubes 208. The inner coil 216 can be free floating or the outer tube 208 can be reflowed through it. The inner coil 216 cand be replaced with a braid (not illustrated).

Figure 6 illustrates an example where the distal section 102 can have smaller diameter ΦA (i.e. a lower profile) than the proximal section 106 (ΦC). Thus, the proximal portion 106 can be thicker for greater pushability and handling, with a smaller distal section/tip 102 for atraumatic clot crossing.

Figures 7 and 8 illustrate an example of the increased diameter portion 220 with a stepped, fluted mid-section 222. Fluted mid-section 222 can be used to provide a smooth transition when used with a funnel catheter 300 to help expand the tip 302 of the catheter 300. The flutes 222 can be thin and soft so that they can be collapsed and retrieved through the body 304 of the funnel catheter 300.

In specific, non-limiting, examples:
the length L of the guidewire 100 can be 180 cm;
the length La of the distal portion 102 can be 3-5 cm, as low as 1 mm or as high as 200 mm, two examples: a short 2cm shapeable tip, and another with a long 15cm tip, of which the most distal approximate 2cm is shapeable;
the distal portion (outer) diameter ΦA can be 0.356-0.559 mm (0.014-0.022 inch) and as low as approximately 0.254 mm (0.010 inch) or as high as 0.965 mm (0.038 inch), one range can be about 0.254 mm (0.010 inch) to about 0.457 mm (0.018 inch) range, and examples can be 0.356 mm (0.014 inch);
the proximal portion length Lc can depend on lengths La and Lb, as overall device length L needs to be longer than the catheter 300 it is used with, in one example, to be a few cm longer than the overall length of the funnel catheter 300, which is approximately 130 cm; and
the proximal portion (outer) diameter ΦC can be 0.356-0.559 mm (0.014-0.022 inch), as high as 1.143 mm (0.045 inch) and as low as 0.356 mm (0.014 inch), and examples are about 0.889 mm (0.035 inch).

Turning to examples of the intermediate/increased diameter portion 104, 200, 202, 208, 220:
the length Lb can be 200-300 mm, in one example, about 20cm;
the (outer) diameter ΦB can be 1.651 mm (0.065 inch); and
the (outer) diameter ΦB can depend on the inner diameter (ID) of the catheter 300 that is being used in conjunction with, and can be approximately 0.076 mm (0.003 inch) to 0.152 mm (0.006 inch) less than that ID.

Examples of the device 100 can be used with a large bore intermediate catheter (1.727 mm - 1.829 mm (0.068 inch - 0.072 inch) ID approximately), a superbore catheter (2.032 mm - 2.337 mm (0.080 inch - 0.092 inch) approximately), or a funnel catheter (shaft ID of 1.727 mm - 1.829 mm (0.068 inch - 0.072 inch) approximately).

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. It is intended that each term contemplates its broadest meaning as understood by those skilled in the art and includes all technical equivalents that operate in a similar manner to accomplish a similar purpose without departing from the scope of the invention. It is also to be understood that the mention of one or more steps of a method does not preclude the presence of additional method steps or intervening method steps between those steps expressly identified. Some steps of a method can be performed in a different order than those described herein without departing from the scope of the disclosed technology. Similarly, it is also to be understood that the mention of one or more components in a device or system does not preclude the presence of additional components or intervening components between those components expressly identified. For clarity and conciseness, not all possible combinations have been listed, and such modifications are often apparent to those of skill in the art and are intended to be within the scope of the claims which follow.

## Claims

1. An access guidewire (100), comprising:
a distal portion (102) comprising a distal portion length (La) and a distal portion diameter (ΦA);
an intermediate portion (104), proximal to the distal portion (102), comprising an intermediate portion length (Lb) and an intermediate portion diameter (ΦB); and
a proximal portion (106), proximal of the intermediate portion (104), comprising a proximal portion length (Lc) and a proximal portion diameter (ΦC);
wherein the intermediate portion diameter is greater than the distal portion diameter (ΦA) and the proximal portion diameter (ΦC),
**characterised in that** the intermediate portion length (Lb) is 200 to 300 mm.

## Patentansprüche

1. Zugriffsführungsdraht (100), umfassend:
einen distalen Abschnitt (102), umfassend eine distale Abschnittslänge (La) und einen distalen Abschnittsdurchmesser (ΦA);
einen Zwischenabschnitt (104), proximal zu dem distalen Abschnitt (102), umfassend eine Zwischenabschnittslänge (Lb) und einen Zwischenabschnittsdurchmesser (ΦB); und
einen proximalen Abschnitt (106), proximal des Zwischenabschnitts (104), umfassend eine proximale Abschnittslänge (Lc) und einen proximalen Abschnittsdurchmesser (ΦC);
wobei der Zwischenabschnittsdurchmesser größer als der distale Abschnittsdurchmesser (ΦA) und der proximale Abschnittsdurchmesser (ΦC) ist,
**dadurch gekennzeichnet, dass** die Zwischenabschnittslänge (Lb) 200 bis 300 mm beträgt.

## Revendications

1. Fil guide d'accès (100) comprenant :
une partie distale (102) comprenant une longueur de partie distale (La) et un diamètre de partie distale (ΦA) ;
une partie intermédiaire (104), proximale par rapport à la partie distale (102), comprenant une longueur de partie intermédiaire (Lb) et un diamètre de partie intermédiaire (ΦB) ; et
une partie proximale (106), proximale de la partie intermédiaire (104), comprenant une longueur de partie proximale (Lc) et un diamètre de partie proximale (ΦC) ;
dans lequel le diamètre de la partie intermédiaire est supérieur au diamètre de la partie distale (ΦA) et au diamètre de la partie proximale (ΦC),
**caractérisé en ce que** la longueur de partie intermédiaire (Lb) est comprise entre 200 et 300 mm.
